# EUROPEAN PATENT APPLICATION

(11) **EP 4 843 486 A1**
(43) Date of publication of application: **25.11.2026**
(21) Application number: 25177676.1
(22) Date of filing: 20.05.2025
(51) Int. Cl.: A61B 5/01, A61F 13/00, G01N 27/04

(54) **METHOD OF SENSING A TEMPERATURE DISTRIBUTION AND A MOISTURE DISTRIBUTION IN A MEDICAL DRESSING, AND SENSING ARRANGEMENT**

(71) Applicant: Mölnlycke Health Care AB, 431 21 Mölndal (SE)
(72) Inventor: WIRTHS, Walter, 82024 TAUFKIRCHEN (DE); HAMMERSBERG, Johan, 448 36 FLODA (SE); HERMANSSON, Anders, 435 41 MÖLNLYCKE (SE)
(74) Representative: Kransell & Wennborg KB

(57) **Abstract**

A sensing arrangement (5) for sensing temperature and moisture in a medical dressing (1) is provided. The sensing arrangement (5) comprises a carrier (19) having a first side and a second side opposite the first side (19), a plurality of conductors (25a-d; 27a-d) supported by the carrier (19), and a plurality of temperature sensing elements (29) supported by the carrier (19). Each temperature sensing element (29) exhibits a temperature-dependent resistance and is electrically conductively connected to a unique combination of two conductors (25a-d; 27a-d). For each temperature sensing element (29), a first conductor (25a-d) is electrically conductively connected to a first conductive structure (31) arranged on the second side (23) of the carrier (19), and a second conductor (27a-d) is electrically conductively connected to a second conductive structure (33) arranged on the second side (23) of the carrier (19) and spaced apart from the first conductive structure (31).

## Description

### Field of the Invention

The present invention relates to a method of sensing a temperature distribution and a moisture distribution in a medical dressing, a sensing arrangement for sensing temperature and moisture in a medical dressing, and a medical dressing comprising such a sensing arrangement.

### Background of the Invention

Both the spatial temperature distribution and the moisture level of a wound and/or skin covered by a medical dressing are useful biomarkers that may provide valuable information, for example, about the wound healing process.

It would therefore be desirable to provide for measurement of both a spatial temperature distribution and a moisture level of a wound and/or skin covered by a medical dressing, such as a wound dressing.

### Summary

It is an object of the present invention to provide for measurement of both a spatial temperature distribution and a moisture level of a wound and/or skin covered by a medical dressing, such as a wound dressing.

According to a first aspect of the present invention, it is therefore provided a sensing arrangement for sensing temperature and moisture in a medical dressing, the sensing arrangement comprising: a carrier having a first side and a second side opposite the first side; a plurality of conductors supported by the carrier; and a plurality of temperature sensing elements supported by the carrier, each temperature sensing element of the plurality of temperature sensing elements exhibiting a temperature-dependent resistance, and being electrically conductively connected to a unique combination of two conductors of the plurality of conductors, wherein, for each temperature sensing element of the plurality of temperature sensing elements: a first conductor of the combination of two conductors electrically conductively connected to the temperature sensing element is electrically conductively connected to a first conductive structure arranged on the second side of the carrier; and a second conductor of the combination of two conductors electrically conductively connected to the temperature sensing element is electrically conductively connected to a second conductive structure arranged on the second side of the carrier and spaced apart from the first conductive structure.

In the context of the present application, a "medical dressing" may refer to a structure or assembly configured to be applied to a region of the body for therapeutic, protective, or diagnostic purposes, including but not limited to covering wounds, monitoring physiological parameters, or delivering medications.

The first and second conductive structures are arranged on the second side of the carrier, which may be intended to face an absorbent layer of the medical dressing when the sensing arrangement is included in a medical dressing. The first conductive structure may be arranged on the same side of the carrier as the first conductor, in which case it may be provided in the form of a segment of the first conductor itself, or as a physically separate structure electrically connected to the first conductor. Alternatively, the first conductive structure may be arranged on the opposite side of the carrier relative to the first conductor, in which case it may be electrically connected to the first conductor via a conductive path passing through the carrier, such as a via. The second conductive structure may likewise be arranged on the same side of the carrier as the second conductor, in which case it may be provided in the form of a segment of the second conductor itself, or as a physically separate structure electrically connected to the second conductor. Alternatively, the second conductive structure may be arranged on the opposite side of the carrier relative to the second conductor, in which case it may be electrically connected to the second conductor via a conductive path passing through the carrier, such as a via.

The present invention is based on the realization that information indicative of both the temperature and the moisture level at a given location in a wound dressing can be obtained using electrical measurements involving the conductors connected to a temperature sensing element arranged at the given location. In particular, the present inventors have realized that the temperature at the location can be estimated based on the electrical resistance of the temperature sensing element, and that the moisture level at the location can be estimated based on the capacitance of a capacitor formed by the conductors connected to the temperature sensing element.

Hereby, both temperature and moisture level can be measured at the same location using a compact structure that requires only two conductors per sensing element, which allows spatially resolved monitoring of two clinically relevant parameters using a single sensing arrangement. This may facilitate early detection of wound complications such as infection or maceration, without increasing the complexity or footprint of the sensor integration in the medical dressing.

According to an example configuration, each temperature sensing element may be arranged on the first side of the carrier, which may define a side of the sensing arrangement to be arranged facing a body of a user. This configuration may allow the temperature sensing elements to be brought into closer thermal contact with the body of the user, thereby improving the accuracy of the temperature estimation for each sensing location.

According to an example configuration, for each temperature sensing element, a minimum distance between the first conductive structure and the second conductive structure may be within 0.5 mm to 10 mm. This configuration may provide a well-defined capacitive coupling between the conductors associated with each temperature sensing element, enabling consistent estimation of the moisture level across multiple sensing locations.

According to an example configuration, the first conductive structure may be a first conductive plate having a first surface area greater than 1 mm², and the second conductive structure may be a second conductive plate having a second surface area greater than 1 mm². The conductive plates may be of any suitable shape, such as rectangular or round. A sufficiently large surface area of the conductive plates may increase the moisture-dependent capacitance relative to the parasitic capacitance between the first conductor and the second conductor, thereby improving the sensitivity of the moisture estimation. The smallest lateral dimension of each conductive plate may advantageously be at least five times, and more advantageously at least ten times, a width of the respective first or second conductor to which it is electrically connected.

According to an example configuration, the sensing arrangement may comprise a plurality of non-overlapping pixel areas, and a temperature sensing element of the plurality of temperature sensing elements, the first conductive structure associated with the temperature sensing element, and the second conductive structure associated with the temperature sensing element may be arranged within each pixel area of the plurality of non-overlapping pixel areas. Defining discrete pixel areas in this way may facilitate spatial mapping of temperature and moisture distributions across the medical dressing. This configuration also ensures that each moisture estimation is spatially related to the temperature sensing element of the same pixel area. Each pixel area may, for example, advantageously be about 2 cm by 2 cm or smaller, such as about 1 cm by 1 cm or smaller, to provide sufficient resolution for clinical assessment while maintaining a compact layout suitable for integration into medical dressings of various sizes.

According to an example configuration, the sensing arrangement may further comprise readout circuitry coupled to each conductor of the plurality of conductors, and configured to address each temperature sensing element of the plurality of temperature sensing elements. The readout circuitry may be configured to address the individual temperature sensing elements sequentially, or several temperature sensing elements in parallel.

In other configurations, the sensing arrangement and the readout circuitry may be provided as separate devices. For instance, the sensing arrangement may comprise a readout interface for electrical connection with readout circuitry. In configurations where the sensing arrangement is included in a medical dressing, such a readout interface may be accessible from the outside of the medical dressing when the medical dressing is arranged on the body of the user.

According to an example configuration, a first set of conductors included in the plurality of conductors may extend in parallel with each other in a first direction, and a second set of conductors included in the plurality of conductors may extend in parallel with each other in a second direction. This configuration, in which the first and second conductors cross each other, may provide for a small parasitic capacitance between the first conductor and the second conductor associated with a temperature sensing element, which in turn may increase the sensitivity of the moisture estimation based on the capacitance between the first and second conductive structures, as compared to a configuration in which the first and second conductors extend side by side.

According to an example configuration, at least one tenth of an area defined by two mutually adjacent conductors extending in the first direction and two mutually adjacent conductors extending in the second direction may be open to passage of fluid. This configuration may provide for fluid-permeability of the sensing arrangement by allowing fluid to pass through spaces between the conductors, without requiring openings in the conductors themselves or in the temperature sensing elements.

According to an example configuration, at least one half of a total area of the sensing arrangement may be open to passage of fluid. A high overall degree of fluid-permeability may be particularly beneficial when the sensing arrangement covers a large portion of the surface of a medical dressing, as it may allow unimpeded transport of fluid through the sensing arrangement and into the absorbent components of the dressing.

Fluid-permeability of the sensing arrangement may be beneficial in applications where the sensing arrangement is included in a medical dressing, as it allows passage of fluid, such as wound exudate or vapor, through the sensing arrangement and into an absorbent layer of the dressing. This may reduce the risk of fluid accumulation at the skin surface, and thereby contribute to reduced risk of maceration or skin irritation. **In** addition, fluid-permeability may support the primary function of the medical dressing, which is to absorb and manage bodily fluids, while enabling spatially resolved sensing.

**In** example configurations, fluid-permeability may be achieved by providing the carrier with perforations or other openings between the conductors. Alternatively, the carrier may be implemented using a fluid-permeable material, such as a woven or non-woven textile, a porous film, or a mesh structure. **In** such configurations, the conductors and temperature sensing elements may be supported on the permeable substrate without the need for dedicated perforations, while still allowing fluid to pass through the sensing arrangement.

According to an example configuration, the carrier may comprise a flexible or stretchable substrate, and the plurality of conductors may be supported by the flexible or stretchable substrate. This configuration may allow the sensing arrangement to conform to curved or moving regions of the body, thereby improving comfort for the user and providing for stable contact with the underlying tissue during use.

Examples of flexible substrates include polyimide films used in flexible printed circuit boards, and plastic films suitable for roll-to-roll manufacturing. Examples of stretchable substrates include thermoplastic elastomers and silicone-based materials, such as polyurethane or polydimethylsiloxane (PDMS), which may allow the sensing arrangement to deform elastically with the movement of the skin. These types of substrates may be selected based on the mechanical requirements of the intended application and the desired degree of conformability.

According to an example configuration, for each temperature sensing element, the temperature sensing element, the first conductor, the second conductor, the first conductive structure, and the second conductive structure may be covered by dielectric material. Covering the conductive structures by dielectric material may reduce the risk of electrical short-circuiting in the presence of fluid, thereby providing for improved estimation of the moisture level. In addition, the risk of corrosion of the conductive structures, and accompanying changes to their electrical properties over time, may be reduced. This configuration may also allow a broader range of materials to be used for the conductors and conductive structures, since the materials do not need to be capable of withstanding direct exposure to body fluids.

According to an example configuration, each temperature sensing element of the plurality of temperature sensing elements may be arranged on the first side of the carrier, and for each temperature sensing element, the temperature sensing element may be coated by a first coating provided on the first side of the carrier, and the first conductive structure and the second conductive structure may be coated by a second coating provided on the second side of the carrier. The first and second coatings may be made from the same or from different materials, depending on the functional requirements on each side. For example, the second coating may advantageously exhibit a relatively high dielectric constant to support capacitive moisture sensing, while the first coating may be selected for compatibility with thermal conduction and mechanical flexibility. Suitable coating materials may include polyurethane, silicone-based materials such as PDMS, or parylene-based coatings.

According to an example configuration, each temperature sensing element may be a thermistor. This configuration may provide a compact and cost-efficient solution for sensing temperature, as thermistors are readily available in small form factors and exhibit a well-defined resistance-temperature relationship suitable for localized temperature estimation. Thermistors may be implemented as discrete surface-mounted components or as printed structures formed using thermistor material deposited on the carrier.

The sensing arrangement according to various example configurations described herein may be included in a medical dressing, further comprising a fluid absorbing pad configured to absorb fluid from a body of a user. The integration of the sensing arrangement into the medical dressing may provide for spatially resolved sensing of temperature and moisture levels across the area of the body covered by the dressing, while allowing the medical dressing to maintain its primary fluid management function.

According to an example configuration, the medical dressing may comprise a body contacting layer and a backing layer sandwiching the fluid absorbing pad, and the sensing arrangement may be arranged between the body contacting layer and the backing layer. In such a configuration, the first side of the carrier of the sensing arrangement may face the body contacting layer. This arrangement may allow the temperature sensing elements to be positioned close to the body of the user, while the remainder of the sensing arrangement remains protected within the structure of the medical dressing.

According to an example configuration, the sensing arrangement may be arranged between the body contacting layer and the fluid absorbing pad. This configuration may provide for improved thermal coupling between the temperature sensing elements and the body of the user, while allowing the first and second conductive structures-facing the fluid absorbing pad-to be positioned in close proximity to the fluid. This may provide for more precise estimation of the moisture level, as the moisture-dependent capacitance is influenced directly by the fluid content of the adjacent absorbent material.

According to a second aspect of the present invention, it is provided a method of sensing a temperature distribution and a moisture distribution in a medical dressing, using the sensing arrangement according to any one of the configurations described herein, the method comprising, for each temperature sensing element of the plurality of temperature sensing elements: estimating a temperature of the temperature sensing element based on a measurement of an electrical resistance between the first conductor and the second conductor; and estimating a moisture level related to the temperature sensing element based on the measured electrical resistance, and an evaluation of a discharge profile of a capacitor formed by the first conductor and the second conductor.

A discharge profile is an indication of a development over time of the amount of charge discharged from a capacitor. The discharge profile may be represented by a current or a voltage, and is generally characterized by a time constant given by the product RC, where R is the resistance through which the capacitor discharges, and C is the capacitance of the capacitor. As is well known to one of ordinary skill in the art, the capacitance can be estimated through analysis of the discharge profile when the resistance is known. This, in turn, provides for an estimation of the moisture level influencing the capacitance.

According to an example of the method, for each temperature sensing element of the plurality of temperature sensing elements, estimating the temperature of the sensing element may comprise measuring a steady-state current flowing through the temperature sensing element while connecting a voltage source providing a known voltage between the first conductor and the second conductor, to allow determination of the electrical resistance of the temperature sensing element, and estimating the temperature of the temperature sensing element based on the electrical resistance of the temperature sensing element; and estimating the moisture level may comprise disconnecting the voltage source, measuring a development over time of a voltage between the first conductor and the second conductor after having disconnected the voltage source, and a development over time of a current flowing through the second conductor after having disconnected the voltage source, and estimating the moisture level based on the electrical resistance of the temperature sensing element, the development over time of the voltage between the first conductor and the second conductor, and the development over time of the current flowing through the second conductor.

### Brief Description of the Drawings

These and other aspects of the present invention will now be described in more detail, with reference to the appended drawings showing example embodiments of the invention, wherein:
Fig. 1 is an illustration of an exemplary absorbent article, in the form of a wound dressing, arranged on the arm of a user;
Fig. 2 is an exploded view of the absorbent article in Fig. 1, with an embedded sensing arrangement according to an example embodiment of the present invention;
Fig. 3 is a top-view of a sensing arrangement according to an example of the invention;
Fig. 4 is an enlarged, and partly opened perspective view of one of the pixel areas of the sensing arrangement in Fig. 3;
Fig. 5 is a circuit schematic representation of the sensing arrangement in Fig. 3;
Fig. 6 is a flow-chart illustrating a first method according to an example of the invention;
Fig. 7 is a flow-chart illustrating a second method according to an example of the invention;
Fig. 8 is a schematic illustration of temperature estimation according to an example of the invention; and
Fig. 9 is a schematic illustration of moisture estimation according to an example of the invention.

### Detailed Description of Example Embodiments

The present invention will now be described more fully hereinafter with reference to the accompanying drawings, in which example embodiments of the present invention are shown. The present invention may, however, be embodied in many different forms and should not be construed as limited to the embodiments set forth herein; rather, these embodiments are provided for thoroughness and completeness, and fully convey the scope of the present invention to the skilled person. Like reference characters refer to like elements throughout the figures.

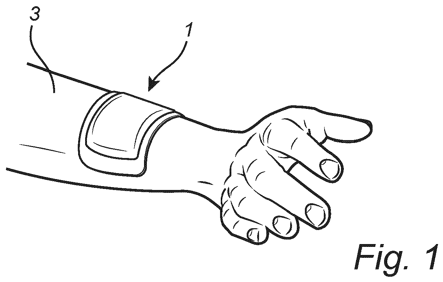

Fig. 1 is an illustration of an exemplary medical dressing 1, in the form of a wound dressing, arranged on the arm 3 of a user. A wound dressing, such as that schematically shown in Fig. 1, may be a particularly interesting application for the sensing arrangement according to embodiments of the present invention, since the temperature and moisture distribution across the wound area may provide an indication of the state of the wound. For instance, the temperature and moisture distribution may provide a qualitative and quantitative indication of an ongoing infection, without the need to remove the wound dressing.

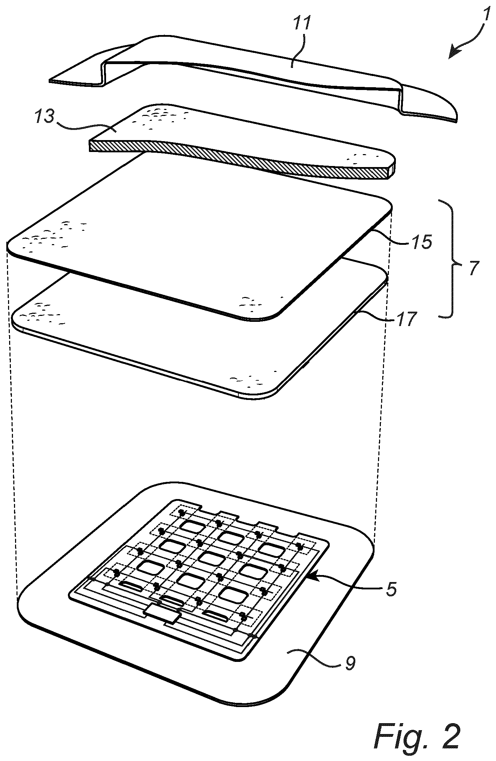

Fig. 2 is an exploded view of the wound dressing 1 in Fig. 1, including an embedded sensing arrangement 5 according to an example embodiment of the present invention. The wound dressing 1 comprises a fluid absorbing pad 7 configured to absorb fluid from a body of a user. In this particular example, the fluid may be wound exudate. The wound dressing 1 comprises a body contacting layer 9 and a backing layer 11 sandwiching the fluid absorbing pad 7 and the sensing arrangement 5. The fluid absorbing pad 7 is, in this example configuration, a layered structure comprising a fluid retention layer 13, a fluid distribution layer 15, and a fluid absorption layer 17. The fluid absorption layer 17 is arranged closest to the body contacting layer 9, the fluid distribution layer 15 is arranged on the fluid absorption layer 17, and the fluid retention layer 13 is arranged on the fluid distribution layer 15. The sensing arrangement 5 may advantageously be arranged between the body contacting layer 9 and the fluid absorption layer 17, so that the sensing arrangement is positioned close to the body of the user while remaining protected within the structure of the wound dressing.

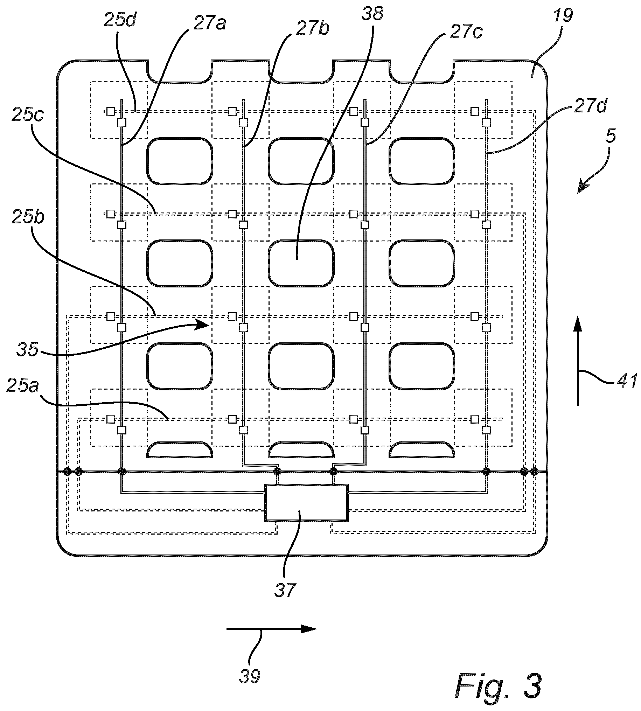

Fig. 3 is a top-view of a sensing arrangement 5 according to an example of the invention. In the example configuration illustrated in Fig. 3, the sensing arrangement 5 comprises a carrier 19, a first set of conductors 25a-d, a second set of conductors 27a-d, a plurality of pixel areas 35, and readout circuitry 37.

In the example configuration shown in Fig. 3, a first set of conductors 25a-d extends in parallel with each other in a first direction 39, and a second set of conductors 27a-d extends in parallel with each other in a second direction 41, intersecting the first direction 39. The carrier 19 may be provided with openings 38 between the conductors. For example, at least one tenth of an area defined by two mutually adjacent conductors of the first set and two mutually adjacent conductors of the second set may be open to passage of fluid.

The carrier 19 may be fluid-permeable in ways other than being provided with dedicated openings 38. For example, the carrier 19 may be implemented using a fluid-permeable material, such as a woven or non-woven textile, a porous film, or a mesh structure. In such configurations, the conductors may be supported directly on the permeable material without the need for dedicated openings. It may also not be necessary for the carrier 19 to be fluid-permeable at all, particularly in configurations where the carrier 19 covers only a relatively small portion of the surface area of the medical dressing. Another variant could be to punch, cut, laser cut, etc. holes into the carrier 19, to create perforations substantially across the entire carrier 19, except where the carrier 19 supports conductors or sensors.

The sensing arrangement 5 may comprise readout circuitry 37 included as part of the sensing arrangement itself. In such configurations, the readout circuitry 37 may be configured to address the conductors 25a-d, 27a-d and perform readout from the sensing arrangement 5. In other configurations, the readout circuitry may be provided in a separate device, and the sensing arrangement 5 may comprise a readout interface for electrical connection with the external readout circuitry. The readout interface may, for example, be arranged at an edge of the sensing arrangement 5 and may be accessible from outside the wound dressing when the wound dressing is applied to a user.

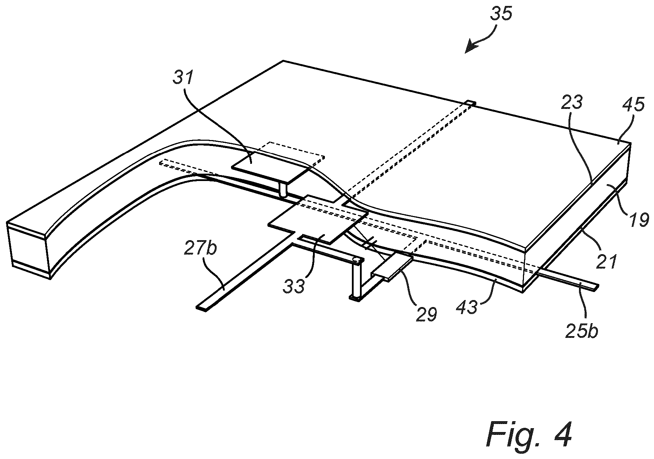

Fig. 4 is an enlarged, and partly opened perspective view of one of the pixel areas 35 of the sensing arrangement 5 in Fig. 3. Like all pixel areas indicated in Fig. 3, the pixel area 35 includes a temperature sensing element 29, a first conductive structure 31, and a second conductive structure 33. The temperature sensing element 29 is electrically conductively connected to a first conductor 25b and a second conductor 27b.

The temperature sensing element 29 exhibits a temperature-dependent resistance and is electrically conductively connected to the first conductor 25b and the second conductor 27b. In the example configuration shown in Fig. 4, the temperature sensing element 29 is arranged on the first side 21 of the carrier 19. The first conductor 25b is electrically conductively connected to the first conductive structure 31, and the second conductor 27b is electrically conductively connected to the second conductive structure 33. The first conductive structure 31 and the second conductive structure 33 are spaced apart from each other and arranged on the second side 23 of the carrier 19.

In the example configuration shown in Fig. 4, the first conductor 25b is arranged on the first side 21 of the carrier 19, and the second conductor 27b is arranged on the second side 23 of the carrier 19. The first conductive structure 31 is electrically connected to the first conductor 25b through a conductive path passing through the carrier 19, such as a via. The second conductive structure 33 is provided in the form of a local widening of the second conductor 27b on the second side 23 of the carrier 19. The second conductor 27b is electrically connected to the temperature sensing element 29 through a via passing through the carrier 19.

In the example configuration shown in Fig. 4, the first conductive structure 31 and the second conductive structure 33 are each provided in the form of square plates having a side length greater than 1 mm, such as about 2 mm. The smallest lateral distance between the first conductive structure 31 and the second conductive structure 33 may advantageously be about 1 mm.

In the example configuration shown in Fig. 4, the temperature sensing element 29, the first conductor 25b, the second conductor 27b, the first conductive structure 31, and the second conductive structure 33 are covered by dielectric material. For each temperature sensing element 29, the temperature sensing element 29 and the first conductor 25b are coated by a first coating 43 provided on the first side 21 of the carrier 19, and the second conductor 27b, the first conductive structure 31, and the second conductive structure 33 are coated by a second coating 45 provided on the second side 23 of the carrier 19. The first coating 43 and the second coating 45 may be made from the same material or from different materials, depending on the functional requirements. For example, the second coating 45 may advantageously exhibit a relatively high dielectric constant to support capacitive sensing, while the first coating 43 may be selected for compatibility with thermal conduction and mechanical flexibility. Suitable materials for the coatings may include polyurethane, silicone-based materials such as PDMS, or parylene-based coatings.

In the example configuration shown in Fig. 4, there is a capacitive coupling between the first conductor 25b and the second conductor 27b. This capacitive coupling includes a parasitic capacitance component, which is mainly defined by the dielectric material of the carrier 19 and the dimensions of the first conductor 25b and the second conductor 27b. In addition to this parasitic component, there is a moisture-dependent capacitance component formed between the first conductive structure 31 and the second conductive structure 33. This capacitance is defined at least in part by the dielectric constant of material located outside the sensing arrangement 5, and varies in response to the local moisture level in the adjacent absorbent material. An estimation of the local moisture level in the vicinity of the first conductive structure 31 and the second conductive structure 33 can thus be made by determining the capacitance of the capacitor formed by the first conductive structure 31 and the second conductive structure 33.

Many other configurations are possible and may be beneficial. For example, both the first conductor 25b and the second conductor 27b may be arranged on the same side of the carrier 19 and spatially separated by a dielectric coating. In such a configuration, the first conductive structure 31 and the second conductive structure 33 may be formed as lateral extensions or widened portions of their respective conductors. The placement, size, and material of the conductors and conductive structures may be adapted based on the specific properties of the medical dressing and the intended application.

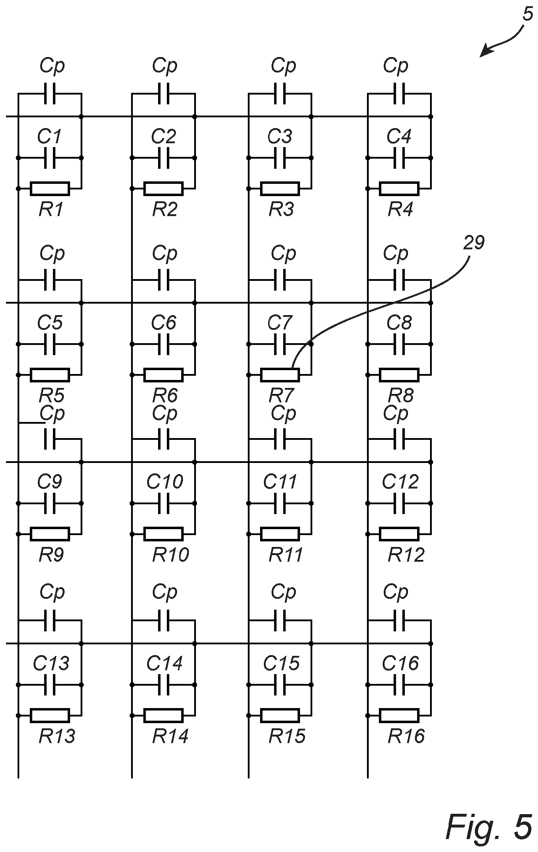

Fig. 5 is a circuit schematic representation of the sensing arrangement 5 shown in Fig. 3, excluding the readout circuitry 37. The schematic illustrates an array of temperature sensing elements 29 arranged in a grid corresponding to the pixel areas 35. Each temperature sensing element 29 exhibits a temperature-dependent resistance, denoted R1-R16 in the schematic. The resistance values R1-R16 may be indicative of the respective temperatures of the temperature sensing elements 29, which may, for example, be implemented as thermistors. For each temperature sensing element 29, the schematic also shows a parasitic capacitance Cp and a moisture-dependent capacitance C1-C16. The parasitic capacitance Cp is independent of the moisture level for all sensing elements. The moisture-dependent capacitance C1-C16 is defined by the dielectric properties of material outside the sensing arrangement 5 and varies in response to the local moisture level in the adjacent absorbent material, when the sensing arrangement 5 is integrated in a medical dressing.

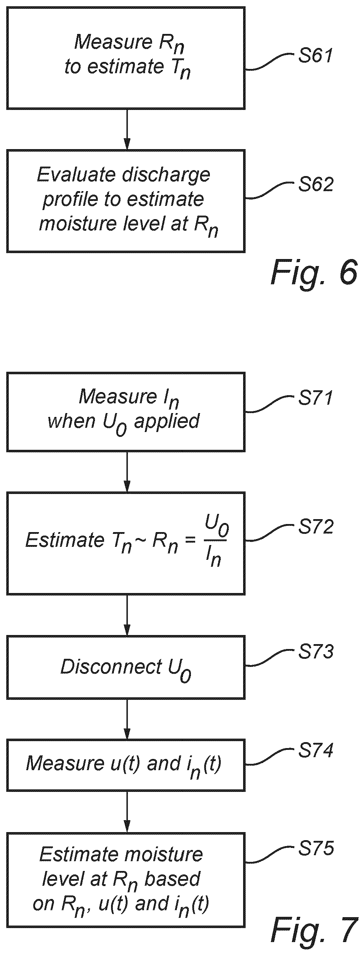

Fig. 6 is a flow-chart illustrating a method of sensing a temperature distribution and a moisture distribution in a medical dressing 1. The method includes, for each temperature sensing element 29 in the sensing arrangement 5, estimating S61 a temperature Tₙ of the temperature sensing element 29 based on a measurement of an electrical resistance Rₙ between the first conductor 25a-d and the second conductor 27a-d, and estimating S62 a moisture level related to the temperature sensing element 29 based on the measured electrical resistance Rₙ, and an evaluation of a discharge profile of a capacitor formed by the first conductor 25a-d and the second conductor 27a-d.

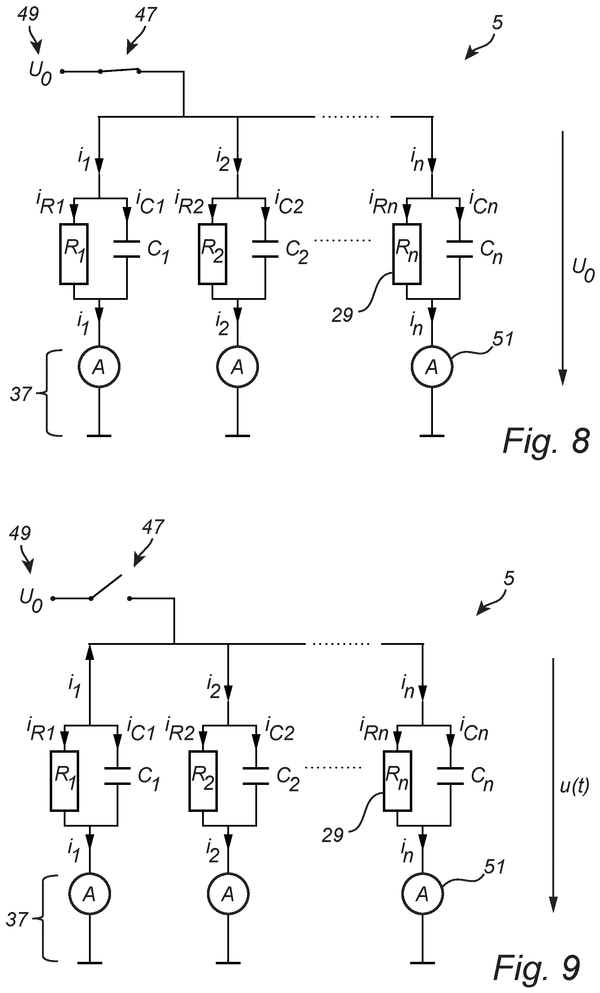

Another example method for estimating a temperature Tₙ and a moisture level for each pixel area 35 of the sensing arrangement 5 will now be described with reference to the flow-chart in Fig. 7 and the supporting illustrations in Fig. 8 and Fig. 9. For ease of illustration, the parasitic capacitance Cp and the moisture-dependent capacitance shown in Fig. 5 have been combined into a total capacitance Cₙ in Fig. 8 and Fig. 9.

The method in Fig. 7 includes, for each temperature sensing element 29 in the sensing arrangement 5, measuring S71 a steady-state current Iₙ flowing through the temperature sensing element 29 while connecting a voltage source 49 providing a constant voltage U₀ between the first conductor 25a-d and the second conductor 27a-d. The measured current Iₙ allows determination of the electrical resistance Rₙ of the temperature sensing element 29.

As schematically illustrated in Fig. 8, the temperature sensing elements 29 in one exemplary row of the sensing arrangement 5 may be connected to a common row conductor (such as the above-mentioned first conductor 25b), while each column conductor (such as the above-mentioned second conductor 27b) is individually connected to a corresponding temperature sensing element 29. A switch 47 may be closed to connect a voltage source 49, providing a constant voltage U₀, to the selected row conductor. This applies the same voltage to one terminal of each temperature sensing element 29 connected to the row conductor. A current sensor 51, schematically indicated by 'A' in Fig. 8, is connected to each column conductor to measure the current Iₙ passing through temperature sensing element 29 connected to that column conductor. The voltage source 49 and the current sensors 51 may be comprised in the readout circuitry 37. The current sensors 51 may, for example, be implemented using operational amplifiers.

Based on the measured current Iₙ and the known applied voltage U₀, the electrical resistance Rₙ of the temperature sensing element 29 can be determined. The temperature Tₙ of the temperature sensing element 29 is then estimated S72 performed based on the resistance value Rₙ. The temperature sensing element 29 may, for example, be a thermistor having a well-defined and monotonic resistance-temperature characteristic, allowing the resistance Rₙ to be converted into a corresponding temperature value Tₙ using calibration data or an analytical model.

After the resistance Rₙ has been determined, the method includes disconnecting S73 the voltage source 49 from the row conductor by opening the switch 47, as illustrated in Fig. 9. This action initiates the discharge of each capacitor formed by the first conductor and the second conductor associated with a respective temperature sensing element 29.

Following disconnection S73 of the voltage source 49, a development over time of a voltage u(t) between the first conductor and the second conductor, and a development over time of a current iₙ(t) flowing through the second conductor, are measured S74. Both the voltage u(t) and the current iₙ(t) are time-varying quantities that decrease with time as a result of the capacitive discharge initiated by opening the switch 47.

With continued reference to Fig. 9, the voltage u(t) between the first and second conductors associated with each temperature sensing element 29 may be measured simultaneously with the current iₙ(t) flowing through the second conductor. This synchronized measurement may be achieved, for example, by employing readout circuitry 37 that includes analog-to-digital converters capable of simultaneous sampling.

Finally, a moisture level related to each temperature sensing element 29 may be estimated S75 based on the electrical resistance Rₙ of the temperature sensing element 29, the development over time of the voltage u(t) between the first conductor and the second conductor, and the development over time of the current iₙ(t) flowing through the second conductor. The capacitance between the row conductor (first conductor) and the column conductor (second conductor) may be determined based on the resistance Rₙ and the measured values of u(t) and iₙ(t). The resulting capacitance may provide an indication of the dielectric constant of the material located between the row conductor and the column conductor. A parasitic capacitance component, which is independent of the moisture level, may be measured and/or calculated in advance and subtracted from the total capacitance to isolate the moisture-dependent contribution.

The total capacitance Cₙ associated with each temperature sensing element 29 may be estimated based on the known resistance Rₙ, the time-dependent voltage u(t) across the capacitor, and the time-dependent discharge current iₙ(t) measured through the second conductor. Since all temperature sensing elements 29 in the addressed row are connected to the same row conductor (first conductor), the voltage u(t) is the same for each of the capacitors. The current iₙ(t), which represents the discharge current from each individual capacitor, is measured independently for each column conductor (second conductor).

The capacitance Cₙ of each individual capacitor can then be determined using the following relation: ${C}_{n} = RC \left(\frac{1}{{R}_{n}}-\frac{{i}_{n} \left(t\right)}{u \left(t\right)}\right) ,$ where RC may be determined from the relation below and an observation over time of u(t). $u \left(t\right) = {U}_{0} {e}^{- \frac{t}{RC}}$

Multiple rows and columns may typically be present in the final setup. To reduce the occurrence of cross-talk, it may be beneficial to follow these guidelines:
- Only one row switch 47 may be closed at a time.
- The capacitors of all rows with open row switches should be discharged before reading the voltage of the row with the closed row switch.
- Care should be taken to prevent potential differences between columns.

It will be appreciated that many variations and modifications of the embodiments described above are possible. For example, the number and layout of the conductors, the physical arrangement of the temperature sensing elements, the structure of the coatings, and the specific readout and measurement techniques may be adapted based on application requirements. The invention is not limited to the specific examples shown, but may be embodied in a variety of forms within the scope of the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1. A sensing arrangement (5) for sensing temperature and moisture in a medical dressing (1), the sensing arrangement (5) comprising:
a carrier (19) having a first side and a second side opposite the first side;
a plurality of conductors (25a-d; 27a-d) supported by the carrier (19); and
a plurality of temperature sensing elements (29) supported by the carrier (19), each temperature sensing element (29) of the plurality of temperature sensing elements exhibiting a temperature-dependent resistance, and being electrically conductively connected to a unique combination of two conductors of the plurality of conductors (25a-d; 27a-d),
wherein, for each temperature sensing element (29) of the plurality of temperature sensing elements:
a first conductor (25a-d) of the combination of two conductors electrically conductively connected to the temperature sensing element (29) is electrically conductively connected to a first conductive structure (31) arranged on the second side (23) of the carrier (19); and
a second conductor (27a-d) of the combination of two conductors electrically conductively connected to the temperature sensing element (29) is electrically conductively connected to a second conductive structure (33) arranged on the second side (23) of the carrier (19) and spaced apart from the first conductive structure (31).

2. The sensing arrangement (5) of claim 1, wherein each temperature sensing element (29) of the plurality of temperature sensing elements is arranged on the first side (21) of the carrier (19).

3. The sensing arrangement (5) of claim 1 or 2, wherein, for each temperature sensing element (29) of the plurality of temperature sensing elements, a minimum distance between the first conductive structure (31) and the second conductive structure (33) is within 0.5 mm - 10 mm.

4. The sensing arrangement (5) of any one of the preceding claims, wherein the first conductive structure (31) is a first conductive plate having a first surface area being greater than 1 mm², and the second conductive structure (33) is a second conductive plate having a second surface area being greater than 1 mm².

5. The sensing arrangement (5) of any one of the preceding claims, wherein:
the sensing arrangement (5) comprises a plurality of non-overlapping pixel areas (35); and
a temperature sensing element (29) of the plurality of temperature sensing elements, the first conductive structure (31) associated with the temperature sensing element (29), and the second conductive structure (33) associated with the temperature sensing element (29) are arranged within each pixel area (35) of the plurality of non-overlapping pixel areas.

6. The sensing arrangement (5) of any one of the preceding claims, further comprising readout circuitry (37) coupled to each conductor (25a-d; 27a-d) of the plurality of conductors, and configured to address each temperature sensing element (29) of the plurality of temperature sensing elements.

7. The sensing arrangement (5) of any one of the preceding claims, wherein:
a first set of conductors (25a-d) included in the plurality of conductors extend in parallel with each other in a first direction (39); and
a second set of conductors (27a-d) included in the plurality of conductors extend in parallel with each other in a second direction (41).

8. The sensing arrangement (5) of claim 7, wherein at least one tenth of an area defined by two mutually adjacent conductors extending in the first direction (39) and two mutually adjacent conductors extending in the second direction (41) is open to passage of fluid.

9. The sensing arrangement (5) of any one of the preceding claims, wherein at least one half of a total area of the temperature sensing arrangement (5) is open to passage of fluid.

10. The sensing arrangement (5) of any one of the preceding claims, wherein the carrier (19) comprises:
a flexible or stretchable substrate; and
the plurality of conductors (25a-d; 27a-d) are supported by the flexible or stretchable substrate.

11. The sensing arrangement (5) of any one of the preceding claims,
wherein, for each temperature sensing element (29), the temperature sensing element (29), the first conductor (25a-d), the second conductor (27a-d), the first conductive structure (31), and the second conductive structure (33) are covered by dielectric material.

12. A medical dressing (1) comprising:
a fluid absorbing pad (7) configured to absorb fluid from a body (3) of a user; and
the sensing arrangement (5) of any one of the preceding claims.

13. The medical dressing (1) of claim 12, wherein:
the medical dressing (1) comprises a body contacting layer (9) and a backing layer (11) sandwiching the fluid absorbing pad (7); and
the sensing arrangement (5) is arranged between the body contacting layer (9) and the backing layer (11),
wherein the first side of the carrier (19) of the sensing arrangement (5) faces the body contacting layer (9).

14. A method of sensing a temperature distribution and a moisture distribution in a medical dressing (1), using the sensing arrangement (5) of any of claims 1-11, the method comprising, for each temperature sensing element (29) of the plurality of temperature sensing elements:
estimating (S61) a temperature of the temperature sensing element based on a measurement of an electrical resistance between the first conductor (25a-d) and the second conductor (27a-d); and
estimating (S62) a moisture level related to the temperature sensing element based on the measured electrical resistance, and an evaluation of a discharge profile of a capacitor formed by the first conductor (25a-d) and the second conductor (27a-d).

15. The method of claim 14, wherein, for each temperature sensing element (29) of the plurality of temperature sensing elements:
estimating the temperature of the sensing element comprises:
measuring (S71) a steady-state current flowing through the temperature sensing element (29) while connecting a voltage source providing a known voltage between the first conductor (25a-d) and the second conductor (27a-d), to allow determination of the electrical resistance of the temperature sensing element (29); and
estimating (S72) the temperature of the temperature sensing element (29) based on the electrical resistance of the temperature sensing element (29); and
estimating the moisture level comprises:
disconnecting (S73) the voltage source;
measuring (S74) a development over time of a voltage between the first conductor (25a-d) and the second conductor (27a-d) after having disconnected the voltage source, and a development over time of a current flowing through the second conductor (27a-d) after having disconnected the voltage source; and
estimating (S75) the moisture level based on the electrical resistance of the temperature sensing element (29), the development over time of the voltage between the first conductor (25a-d) and the second conductor (27a-d), and the development over time of the current flowing through the second conductor (27a-d).
